# FASCICULE DE BREVET EUROPEEN

(11) **EP 1 010 698 B1**
(45) Date de publication et mention de la délivrance du brevet: **27.03.2002**
(21) Numéro de dépôt: 99403133.4
(22) Date de dépôt: 14.12.1999
(51) Int. Cl.: C07D 339/04, A61K 31/385, C07D 413/12

(54) **Nouveaux dérivés de 1,2-dithiolane, leur procédé de préparation et les compositions pharmaceutiques qui les contiennent.**
1,2-Dithiolan Derivate, Verfahren zu deren Herstellung und diese enthaltende pharmazeutische Zusammenstellungen
1,2-dithiolane derivatives, their process of preparation and pharmaceutical compositions containing them

(30) Priorité: 15.12.1998 FR 9815821
(43) Date de publication de la demande: 21.06.2000
(73) Titulaire: LES LABORATOIRES SERVIER, 92200 Neuilly sur Seine (FR)
(72) Inventeur: Golstein, Solo, 92150 Suresnes (FR); Guillonneau, Claude, 92140 Clamart (FR); Charton, Yves, 92330 Sceaux (FR); Lestage, Pierre, 78170 La Celle Saint Cloud (FR); Lockhart, Brian, 78290 Croissy sur Seine (FR)

(56) Documents cités:
- EP-A- 0 869 126
- FR-A- 2 707 983

## Description

La présente invention concerne de nouveaux dérivés de 1,2-dithiolane, leur procédé de préparation et les compositions pharmaceutiques qui les contiennent.

Ces nouveaux composés constituent des antioxydants, piégeurs d'espèces oxygénées réactives, capables de s'opposer à des "stress oxydants" au niveau cérébral.

Selon la théorie radicalaire du vieillissement de Hartman, les agressions oxydantes successives créent des conditions de "stress oxydant", c'est-à-dire un déséquilibre entre les systèmes protecteurs en faveur des pro-oxydants. Ces agressions conduisent à de nombreuses modifications moléculaires, notamment des lipides membranaires polyinsaturés, des protéines et des acides nucléiques. Les organismes, humain et animal, disposent de différents mécanismes de défense agissant en synergie. Ces mécanismes sont de nature enzymatique (superoxyde dismutase, catalase, glutathion peroxydase) ou non enzymatique (comme les vitamines E ou C qui permettent physiologiquement de contrôler l'action des radicaux libres). Mais, avec l'âge, cette protection devient moins efficace, voire inefficace, en particulier du fait de l'inaction oxydative de nombreuses enzymes dont celles participant à ces mécanismes de défense. Ainsi, dans certaines affections liées à la sénescence, telles que l'athérosclérose, la cataracte, le diabète non insulino-dépendant, le cancer ou les maladies neurodégénératives chroniques, de nombreux travaux ont pu montrer qu'elles étaient associées à ces conditions de "stress oxydant".

Le système nerveux central est particulièrement sensible au "stress oxydant" en raison de sa haute consommation d'oxygène, des niveaux relativement faibles de ses défenses antioxydantes, et de la forte teneur en fer de certaines aires cérébrales. Ceci explique que le "stress oxydant" puisse être l'un des facteurs étiologiques principaux du vieillissement cérébral, ainsi que des maladies neurodégénératives chroniques, particulièrement la maladie d'Alzheimer et les neurodégénérescences des ganglions de la base.

La demande de brevet EP 0 869 126 décrit des dérivés d'acide thioctique possédant des propriétés antioxydante grâce à leur action sur la gluthatione réductase.

Les composés de la présente invention, outre le fait qu'ils soient nouveaux, se sont révélés être des antioxydants plus puissants que l'acide thioctique. Ces propriétés caractéristiques rendent donc les composés de l'invention potentiellement utiles pour le traitement et la prévention des pathologies, d'une part liées au vieillissement et en particulier au vieillissement cérébral, et d'autre part dues au stress oxydant.

Les composés de l'invention constituent ainsi des agents thérapeutiques capables de s'opposer aux troubles cognitifs associés au vieillissement cérébral ou aux maladies neurodégénératives, ainsi qu'à la mort neuronale associée non seulement aux maladies neurodégénératives aiguës comme par exemple l'ischémie et l'épilepsie, mais encore à celle associée aux maladies neurodégénératives progressives, comme par exemple la maladie d'Alzheimer, la maladie de Pick ou les neurodégénérescences des ganglions de la base.

Les composés de la présente invention, outre le fait qu'ils soient nouveaux, se sont révélés être des antioxydants plus puissants que l'acide thioctique.

Plus spécifiquement, les composés de la présente invention concernent les composés de formule (I) : 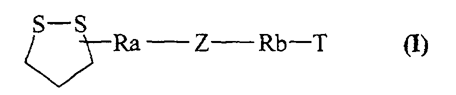 dans laquelle :
- Ra: représente un groupement alkylène (C₁-C₈) linéaire ou ramifié,
- Rb: représente une liaison simple ou un groupement alkylène (C₁-C₆) linéaire ou ramifié,
- Z: représente un groupement :
- thiocarbamate 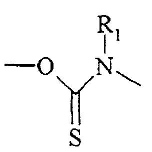 dans lequel l'atome d'oxygène est relié au groupement Ra,
   et R₁ représente un atome d'hydrogène, un groupement alkyle (C₁-C₆) linéaire ou ramifié, aryle ou arylalkyle (C₁-C₆) linéaire ou ramifié.
- ou thioamide 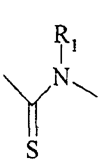 dans lequel le groupement thiocarbonyle est relié au groupement Ra,
   et R₁ est tel que défini précédemment,
- T: représente un groupement : 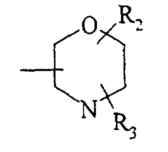 dans lequel R₂ et R₃, identiques ou différents, indépendamment l'un de l'autre, représentent un groupement choisi parmi :
atome d'hydrogène, groupement alkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, et aminoalkyle (C₁-C₆) linéaire ou ramifié (la partie amino étant éventuellement substitué par un ou deux groupements, identiques ou différents, choisis parmi alkyle (C₁-C₆) linéaire ou ramifié, aryle et arylalkyle (C₁-C₆) linéaire ou ramifié),
leurs isomères, ainsi que leurs sels d'addition à un acide ou à une base pharmaceutiquement acceptable.

Par cycloalkyle, on entend un groupement mono ou bicyclique, saturé ou insaturé, comportant de 3 à 8 atomes de carbone, chacun de ces groupements étant éventuellement substitués, de façon identique ou différente, par un ou plusieurs groupements choisis parmi atome d'halogène, groupement hydroxy, alkyle (C₁-C₆) linéaire ou ramifié, alkoxy (C₁-C₆) linéaire ou ramifié, et amino (lui-même éventuellement substitué par un ou deux groupements, identiques ou différents, alkyle (C₁-C₆) linéaire ou ramifié).

Par hétérocycloalkyle, on entend un groupement cycloalkyle dans lequel un ou deux atomes de carbone sont remplacés par un hétéroatome, choisi de façon identique ou différente, parmi azote, oxygène ou soufre.

Par aryle, on entend un groupement phényle, naphtyle, indényle, tétrahydronaphtyle, dihydronaphtyle, indanyle, chacun de ces groupements étant éventuellement substitué de façon identique ou différente, par un ou plusieurs groupements choisis parmi atome d'halogène, groupement alkyle (C₁-C₆) linéaire ou ramifié, hydroxy, alkoxy (C₁-C₆) linéaire ou ramifié, amino, mono ou dialkylamino (C₁-C₆) linéaire ou ramifié, carboxy, et alkoxycarbonyle (C₁-C₆) linéaire ou ramifié.

Par hétéroaryle, on entend un groupement aryle dans lequel un ou deux atomes de carbone sont remplacés par un hétéroatome, choisi de façon identique ou différente, parmi oxygène, azote et soufre.

Par isomères, on entend les isomères optiques

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels le cycle 1,2-dithiolane est substitué en position 3.

Le groupement Z préféré des composés de l'invention est le groupement thiocarbamate 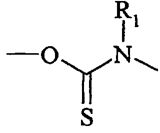 dans lequel R₁ est tel que défini dans la formule (I).

Selon une variante avantageuse, les composés préférés de l'invention sont les composés de formule (I) dans laquelle Z représente un groupement thiocarbamate

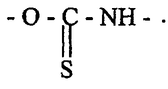

Le groupement T préféré des composés de l'invention est le groupement 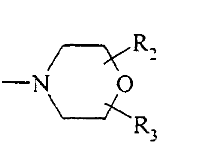 dans lequel R₂ et R₃ sont tels que définis dans la formule (I) et d'une façon avantageuse R₂ et R₃ représentent chacun un atome d'hydrogène.

Les composés préférés de l'invention sont les composés de formule (I) correspondant au :
- chlorhydrate du 5-(1,2-dithiolan-3-yl)pentyl-N-(2-morpholinoéthyl)thiocarbamate,
- chlorhydrate du 5-[(*3S*)-1,2-dithiolan-3-yl]pentyl-N-(2-morpholinoéthyl)thiocarbamate,
- chlorhydrate du 5-[(3*R*)-1,2-dithiolan-3-yl]pentyl-N-(2-morpholinoéthyl)thiocarbamate,
- chlorhydrate du 5-(1,2-dithiolan-3-yl)pentyl-N-(3-morpholinopropyl)thiocarbamate,
- et au chlorhydrate du 5-(1,2-dithiolan-4-yl)pentyl-N-(2-morpholinoéthyl)thiocarbamate.

Les isomères ainsi que les sels d'addition à un acide ou à une base pharmaceutiquement acceptable des composés préférés font partie intégrante de l'invention.

L'invention s'étend également au procédé de préparation des composés de formule (I) caractérisé en ce qu'on utilise comme produit de départ :
- *soit un composé de formule (II) :* 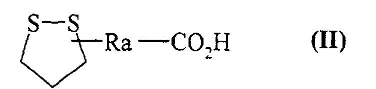 dans laquelle Ra est tel que défini dans la formule (I), composé de formule (II),
   que l'on traite en milieu basique avec un composé de formule (III) : 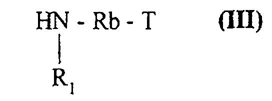 dans laquelle R₁, Rb et T sont tels que définis dans la formule (I),
   pour conduire aux composés de formule (IV) : 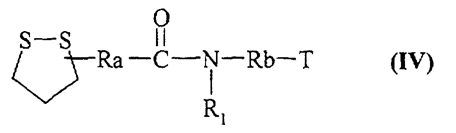 dans laquelle Ra, Rb, R₁ et T sont tels que définis précédemment,
   que l'on soumet au réactif de Lawesson, pour conduire aux composés de formule (I/a), cas particulier des composés de formule (I): 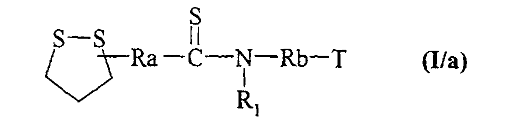 dans laquelle Ra, Rb, R₁ et T sont tels que définis précédemment,
- *soit un composé de formule (V) :* 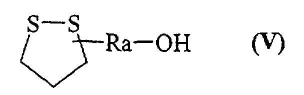 dans laquelle Ra a la même signification que dans la formule (I),
   que l'on fait réagir, en présence de bis(tributylétain)oxyde, avec un thioisocyante de formule (VI) :

   S=C=N-Rb-T (VI)

   dans laquelle Rb et T ont la même signification que dans la formule (I),
   pour conduire aux composés de formule (I/b), cas particulier des composés de formule (I): 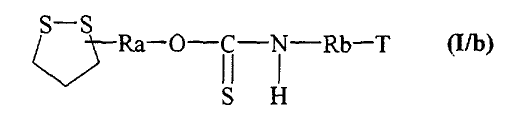 dans laquelle Ra, Rb, et T sont tels que définis précédemment,
   composés de formule (I/b) que l'on traite, si on le souhaite, par un composé de formule (VII) :

   R₁ - X (VII)

   dans laquelle R₁ est tel que défini dans la formule (I) et X représente un atome d'halogène.
   pour conduire aux composés de formule (I/c), cas particulier des composés de formule (I): 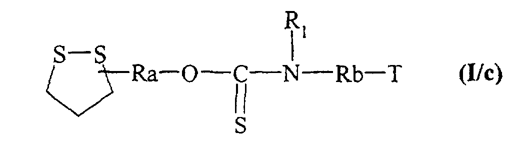 dans laquelle Ra. Rb, R₁ et T sont tels que définis précédemment,
les composés (I/a) à (I/c) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II), (III), (V), (VI) et (VII) sont soit des composés commerciaux, soit obtenus selon des méthodes connues et classiques de la synthèse organique.

La présente invention a également pour objet les compositions pharmaceutiques renfermant comme principe actif au moins un composé de formule (I), ses isomères optiques ou un de ses sels d'addition à un acide ou à une base pharmaceutiquement acceptable, seul ou en combinaison avec un ou plusieurs excipients ou véhicules inertes, non toxiques et pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels et s'échelonne de 10 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus.

### EXEMPLE 1: Chlorhydrate de 5-(1,2-Dithiolan-3-yl)-N[2-(4-morpholinyl) éthyl]pentanethioamide

### Stade A : 5-(1,2-Dithiolan-3-yl)-N[2-(4-morpholinyl)éthyl]pentanamide

A une solution de 6,2 g d'acide thioctique dans 180 ml de tétrahydrofurane sont additionnés à température ambiante, 4,5 g d'hydroxybenzotriazole, 10,5 g de 2-(1H-benzotriazole-1-yl)-1,1,3,3-tétraméthyluronium tétrafluoroborate, 7,8 g de diisopropyléthstlamine et 3,9 g de 2-morpholino-1-éthylamine. Après 20 heures, le milieu réactionnel est concentré sous pression réduite. Le résidu est repris à l'acétate d'éthyle, lavé puis séché sur Na₂SO₄. Après concentration, sous pression réduite, on obtient 8,4 g du produit attendu.

### Stade B : Chlorhydrate de 5-(1,2-Dithiolan-3-yl)-N[2-(4-morpholinyl) éthyl]pentanethioamide

Une solution de 8,3 g du composé obtenu dans le stade A, 5,3 g du réactif de Lawesson, dans 250 ml de toluène est portée à 80°C pendant 4 heures. Le milieu réactionnel est ensuite refroidi puis extrait par une solution d'acide chlorhydrique 1N. La phase aqueuse est alors alcalinisée par addition de Na₂CO₃ puis extraite à l'acétate d'éthyle. Les phases organiques rassemblées sont séchées puis concentrées sous pression réduite. Le résidu est alors repris dans l'éthanol, acidifié puis concentré de nouveau. Après addition d'éther, trituration et filtration du résidu final, on isole le produit attendu.

| *Microanalyse élémentaire :* | | | | | |
|---|---|---|---|---|---|
| | *C* | *H* | *N* | *S* | *Cl* |
| *% calculé* | *45,32* | *7,33* | *7,55* | *25,93* | *9,56* |
| *% trouvé* | *45,24* | *7,23* | *7,61* | *25,75* | *9,48* |

*Point de fusion : 98-100°C*

### EXEMPLE 2: Chlorhydrate du 5-(1,2-Dithiolan-3-yl)pentyl-N-(2-morpholinoéthyl)thiocarbamate

Une solution de 9,6 g de 5-(1,2-dithiolan-3-yl)-1-pentanol et 30 g de bis(tributylétain)oxyde dans 400 ml de toluène est portée au reflux pendant 20 heures, puis refroidit à 60°C. On ajoute alors 1,2 équivalents de 2-(morpholin-4-yl)1-éthylisocyanate et le milieu réactionnel est maintenu 22 heures à 60°C. Après refroidissement, on extrait par une solution d'acide chlorhydrique 1N. La phase aqueuse est ensuite neutralisée puis extraite à l'acétate d'éthyle et les phases organiques rassemblées sont séchées puis concentrées sous pression réduite. Une chromatographie sur gel de silice (dichlorométhane/tétrahydrofurane / 95/5) permet d'isoler le produit attendu.

| *Microanalyse élémentaire :* | | | | | |
|---|---|---|---|---|---|
| | *C* | *H* | *N* | *S* | *Cl* |
| *% calculé* | *43,94* | *7,37* | *6,83* | *23,46* | *8,65* |
| *% trouvé* | *43,70* | *7,55* | *7,05* | *21,31* | *9,34* |

*Point de fusion : 105-110°C*

### EXEMPLE 3: Chlorhydrate du (R)-5-(1,2-Dithiolan-3-yl)pentyl-N-(2-morpholinoéthyl)thiocarbamate

On procède comme dans l'exemple 2 en utilisant comme substrat le (*R*)-5-(1,2-dithiolan-3-yl)-1-pentanol.

| *Microanalyse élémentaire :* | | | | | |
|---|---|---|---|---|---|
| | *C* | *H* | *N* | *S* | *Cl* |
| *% calculé* | *44,92* | *7,29* | *6,98* | *23,98* | *8,84* |
| *% trouvé* | *44,72* | *7,27* | *6,90* | *24,19* | *8,79* |

*Point de fusion : 108-110°C*

### EXEMPLE 4: Chlorhydrate du (S)-5-(1,2-Dithiolan-3-yl)pentyl-N-(2-morpholinoéthyl)thiocarbamate

On procède comme dans l'exemple 2 en utilisant comme substrat le (S)-5-(1,2-dithiolan-3-yl)-1-pentanol.

| *Microanalyse élémentaire :* | | | | | |
|---|---|---|---|---|---|
| | *C* | *H* | *N* | *S* | *Cl* |
| *% calculé* | *44,92* | *7,29* | *6,99* | *8,84* | *23,98* |
| *% trouvé* | *44,08* | *7,49* | *6,88* | *8,84* | *24,00* |

*Point de fusion : 112-116°C*

### EXEMPLE 5: Chlorhydrate du 5-(1,2-Dithiolan-3-yl)pentyl-N-(3-morpholinopropyl)thiocarbamate

On procède comme dans l'exemple 2 en utilisant comme réactif le 3-(morpholin-4-yl)-1-propylthioisocyanate.

| *Microanalyse élémentaire :* | | | | | |
|---|---|---|---|---|---|
| | *C* | *H* | *N* | *S* | *Cl* |
| *% calculé* | *46,30* | *7,53* | *6,75* | *23,17* | *8,54* |
| *% trouvé* | *46,24* | *7,41* | *6,65* | *22,76* | *8,47* |

*Point de fusion : 86-88°C*

### EXEMPLE 6: Chlorhydrate du 5-(1,2-Dithiolan-4-yl)pentyl-N-(2-morpholinoéthyl)thiocarbamate

On procède comme dans l'exemple 2 en utilisant comme substrat le 5-(1,2-dithiolan4-yl)-1-pentanol.

| *Microanalyse élémentaire :* | | | | | |
|---|---|---|---|---|---|
| | *C* | *H* | *N* | *S* | *Cl* |
| *% calculé* | *44,92* | *7,29* | *6,98* | *23,98* | *8,81* |
| *%trouvé* | *44,50* | *7,56* | *7,27* | *24,17* | *8,84* |

*Point de fusion : 80-85°C*

### EXEMPLE 7: 5-(1,2-Dithiolan-3-yl)pentyl-N[2-(3,5-diméthyl)morpholinoéthyl] thiocarbamate

On procède comme dans l'exemple 2 en utilisant comme réactif le 4-(2-isothiocyanatoéthyl)-3,5-diméthylmorpholine.

### EXEMPLE 8: N-(2-(4-Benzyl-2-morpholinyl)éthyl)-5-(1,2-dithiolan-3-yl)pentanethioamide

On procède comme dans l'exemple 1 des stades A à B en utilisant comme réactif au stade A la 2-(4-benzyl-2-morpholinyl)éthylamine.

### ETUDE PHARMACOLOGIQUE DES COMPOSES DE L'INVENTION

### EXEMPLE 9 : Test de cytotoxicité à l'acide L-homocystéique

Ce test permet d'évaluer *in vitro* les propriétés neuroprotectrices des composés testés, en déterminant leur capacité à s'opposer à la cytotoxicité provoquée par une exposition des cellules murines hippocampiques HT 22 à l'acide L-homocystéique *(Neuron;* 2, 1989, 1547-1885).

Des cellules hippocampiques murines HT 22 en culture sont préincubées pendant 1 heure en présence de sept concentrations (5, 10, 25, 50, 75, 100 et 200 µM) de l'agent « anti-oxydant » étudié. Les cultures cellulaires sont ensuite exposées pendant 48 heures à 2 mM d'acide L-homocystéique en présence ou en absence d'agents anti-oxydants. La cytotoxicité est évaluée par la méthode de réduction du bromure de 3-(4,5-diméthylthiazol-2-yl)-2,5-diphényl-tétrazolium (*Immunol. Methods*., 1983, 65, 55-63). Les résultats sont exprimés par la PC 50, concentration représentant 50 % de protection par rapport à la cytotoxicité mesurée dans les cultures cellulaires en absence d'agents anti-oxydants. Lors de ce test, le composé de l'exemple 2 présente une PC 50 de 15,5 µM.

### EXEMPLE 10: Dosage de la peroxydation lipidique

Les membranes de cortex de souris mâle NMRI (lg/20ml dans du Tris HCI 20mM pH 7,4) sont préincubées pendant 15 min à 37°C avec l'agent anti-oxydant étudié à la concentration de 5 mM. Ensuite, les membranes sont exposées pendant 15 min en présence de FeSO₄ (100µM)/acide ascorbique (1000µM)/H₂O₂ (1000µM). La réaction est arrêtée avec de l'acide trichloroacétique (20%v/v) à +4°C puis l'échantillon est centrifugé à 1500 g pendant 5 min à +4°C. Ensuite, un volume équivalent (1ml) d'acide thiobarbiturique (0,67%) est ajouté au surnageant et incubé à +100°C pendant 20 min. La réaction est arrêtée sur glace. Cette solution est lue à 532 nm avec un spectrophotomètre (détermination des concentrations en malondialdéhyde, exprimées en TBARS). Une gamme d'étalon de malondialdéhyde (0-40µM) est établie pour chaque expérience. Pour chaque agent anti-oxydant testé, les résultats sont exprimés en pourcentage d'inhibition par rapport au contrôle (FeSO₄/acide ascorbique/H₂O₂) seul. Lors de ce test, le composé de l'exemple 2 présente une inhibition de 90 %.

### EXEMPLE 11: Test de létalité

Ce test permet de déterminer *in vivo* les propriétés neuroprotectrices des composés testés, en mesurant leur capacité à s'opposer à la létalité induite, chez la souris, par une administration intracérébroventriculaire de t-butylhydroperoxyde, agent oxydant capable de provoquer des neurodégénérescences de type apoptotique (*Free radical Biol. Med*., 1993, 15, 195-202 et *Mol. Chem. Neuropathol*., 1991, 26, 95-106).

L'administration intracérébroventriculaire de t-butylhydroperoxyde (1 µl d'une solution à 70 %) provoque une létalité chez la souris mâle NMRI (30-35 g). La létalité est mesurée deux heures après l'administration de t-butylhydroperoxyde et est exprimée en pourcentage de protection par rapport à la létalité chez les animaux ayant reçu le véhicule des agents « anti-oxydants » étudiés. Ceux-ci sont administrés par voie intrapéritonéale 30 minutes avant l'administration de t-butylhydroperoxyde, à la dose de 150 mg/kg. Lors de ce test, le composé de l'exemple 2 fournit 100 % de protection.

### Example 12 : Hyperglycémie induite par l'alloxane chez la souris NMRI.

L'administration intravèineuse d'alloxane (40 mg/kg) provoque chez la souris mâle NMRI (30-35 g; à jeun depuis 18-24 heures) une hyperglycémie mesurée 24 heures après l'administration d'alloxane. L'hyperglycémie est déterminée par dosage du *D*-glucose dans le plasma en pourcentage d'inhibition de l'hyperglycémie chez les animaux ayant reçu l'agent « anti-oxydant » (administré 1 ou 3 heures à 400 mg/kg *per os* avant l'alloxane) par rapport aux animaux traités avec l'alloxane seul. Le composé de l'exemple 2 inhibe aussi l'hyperglycémie à 100 % (administration d'1 heure) et 92 % (administration de 3 heures).

## Revendications

1. Composés de formule (I): 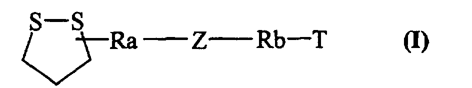 dans laquelle :
Ra représente un groupement alkylène (C₁-C₈) linéaire ou ramifié,
Rb représente une liaison simple ou un groupement alkylène (C₁-C₆) linéaire ou ramifié,
Z représente un groupement :
• thiocarbamate 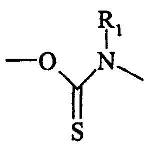 dans lequel l'atome d'oxygène est relié au groupement Ra,
et R₁ représente un atome d'hydrogène, un groupement alkyle (C₁-C₆) linéaire ou ramifié, aryle ou arylalkyle (C₁-C₆) linéaire ou ramifié,
• ou thioamide 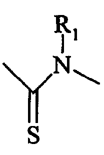 dans lequel le groupement thiocarbonyle est relié au groupement Ra,
et R₁ est tel que défini précédemment,
T représente un groupement : 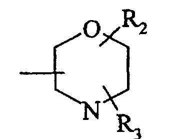 dans lequel R₂ et R₃, identiques ou différents, indépendamment l'un de l'autre, représentent un groupement choisi parmi :
atome d'hydrogène, groupement alkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, et aminoalkyle (C₁-C₆) linéaire ou ramifié, la partie amino étant éventuellement substitué par un ou deux groupements, identiques ou différents, choisis parmi alkyle (C₁-C₆) linéaire ou ramifié, aryle et arylalkyle (C₁-C₆) linéaire ou ramifié,
leurs isomères optiques, ainsi que leurs sels d'addition à un acide ou à une base pharmaceutiquement acceptable,
étant entendu que :
- par cycloalkyle, on comprend un groupement mono ou bicyclique, saturé ou insaturé, comportant de 3 à 8 atomes de carbone, chacun de ces groupements étant éventuellement substitués, de façon identique ou différente, par un ou plusieurs groupements choisis parmi atome d'halogène, groupement hydroxy, alkyle (C₁-C₆) linéaire ou ramifié, alkoxy (C₁-C₆) linéaire ou ramifié, et amino lui-même éventuellement substitué par un ou deux groupements, identiques ou différents, alkyle (C₁-C₆) linéaire ou ramifié,
- par hétérocycloalkyle, on comprend un groupement cycloalkyle dans lequel un ou deux atomes de carbone sont remplacés par un hétéroatome, choisi de façon identique ou différente, parmi azote, oxygène ou soufre,
- par aryle, on comprend un groupement phényle, naphtyle, indényle, tétrahydronaphtyle, dihydronaphtyle, indanyle, chacun de ces groupements étant éventuellement substitué de façon identique ou différente, par un ou plusieurs groupements choisis parmi atome d'halogène, groupement alkyle (C₁-C₆) linéaire ou ramifié, hydroxy, alkoxy (C₁-C₆) linéaire ou ramifié, amino, mono ou dialkylamino (C₁-C₆) linéaire ou ramifié, carboxy, et alkoxycarbonyle (C₁-C₆) linéaire ou ramifié,
- et par hétéroaryle, on comprend un groupement aryle dans lequel un ou deux atomes de carbone sont remplacés par un hétéroatome, choisi de façon identique ou différente, parmi oxygène, azote et soufre.

2. Composés de formule (I) selon la revendication 1 **caractérisé en ce qu'**ils représentent un composé de formule (IA) : 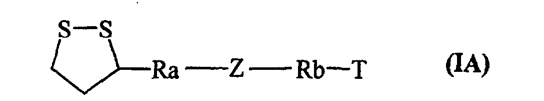 dans laquelle :
Ra représente un groupement alkylène (C₁-C₈) linéaire ou ramifié,
Rb représente une liaison simple ou un groupement alkylène (C₁-C₆) linéaire ou ramifié,
Z représente un groupement :
• thiocarbamate 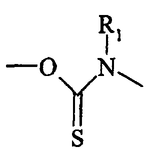 dans lequel l'atome d'oxygène est relié au groupement Ra,
et R₁ représente un atome d'hydrogène, un groupement alkyle (C₁-C₆) linéaire ou ramifié, aryle ou arylalkyle (C₁*-*C₆) linéaire ou ramifié,
• ou thioamide 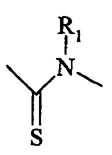 dans lequel le groupement thiocarbonyle est relié au groupement Ra,
et R₁ est tel que défini précédemment,
T représente un groupement : 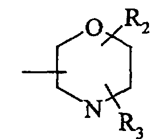 dans lequel R₂ et R₃, identiques ou différents, indépendamment l'un de l'autre, représentent un groupement choisi parmi :
atome d'hydrogène, groupement alkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, et aminoalkyle (C₁-C₆) linéaire ou ramifié, la partie amino étant éventuellement substitué par un ou deux groupements, identiques ou différents, choisis parmi alkyle (C₁-C₆) linéaire ou ramifié, aryle et arylalkyle (C₁-C₆) linéaire ou ramifié,
leurs isomères optiques, ainsi que leurs sels d'addition à un acide ou à une base pharmaceutiquement acceptable.

3. Composés de formule (I) selon la revendication 1 **caractérisés en ce qu'**ils représentent un composé de formule (IB): 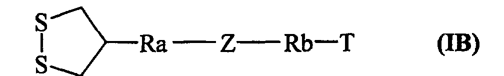 dans laquelle :
Ra représente un groupement alkylène (C₁-C₈) linéaire ou ramifié,
Rb représente une liaison simple ou un groupement alkylène (C₁-C₆) linéaire ou ramifié,
Z représente un groupement :
• thiocarbamate 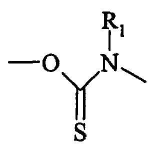 dans lequel l'atome d'oxygène est relié au groupement Ra,
et R₁ représente un atome d'hydrogène, un groupement alkyle (C₁-C₆) linéaire ou ramifié, aryle ou arylalkyle (C₁-C₆) linéaire ou ramifié,
• ou thioamide 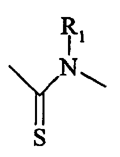 dans lequel le groupement thiocarbonyle est relié au groupement Ra,
et R₁ est tel que défini précédemment,
T représente un groupement : 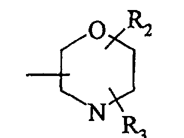 dans lequel R₂ et R₃, identiques ou différents, indépendamment l'un de l'autre, représentent un groupement choisi parmi:
atome d'hydrogène, groupement alkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, et aminoalkyle (C₁-C₆) linéaire ou ramifié, la partie amino étant éventuellement substitué par un ou deux groupements, identiques ou différents, choisis parmi alkyle (C₁-C₆) linéaire ou ramifié, aryle et arylalkyle (C₁-C₆) linéaire ou ramifié,
leurs isomères optiques, ainsi que leurs sels d'addition à un acide ou à une base pharmaceutiquement acceptable.

4. Composés de formule (I) selon l'une quelconque des revendications 1 à 3 **caractérisés en ce que** Z représente un groupement thiocarbamate 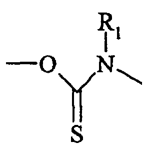 dans lequel R₁ est tel que défini dans la formule (I),
leurs isomères optiques, ainsi que leurs sels d'addition à un acide ou à une base pharmaceutiquement acceptable.

5. Composés de formule (I) selon l'une quelconque des revendications 1 à 4 **caractérisés en ce que** Z représente un groupement thiocarbamate 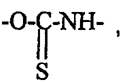 leurs isomères optiques, ainsi que leurs sels d'addition à un acide ou à une base pharmaceutiquement acceptable.

6. Composés de formule (I) selon l'une quelconque des revendications 1 à 3 **caractérisés en ce que** T représente le groupement 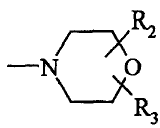 dans lequel R₂ et R₃ sont tels que définis dans la formule (I), leurs isomères optiques, ainsi que leurs sels d'addition à un acide ou à une base pharmaceutiquement acceptable.

7. Composés de formule (I) selon la revendication 6 **caractérisés en ce que** R₂ et R₃, identiques, représentent un atome d'hydrogène, leurs isomères optiques, ainsi que leurs sels d'addition à un acide ou à une base pharmaceutiquement acceptable.

8. Composé de formule (I) selon l'une quelconque des revendications 1 ou 2 qui est le chlorhydrate du 5-(1,2-dithiolan-3-yl)pentyl-N-(2-morpholinoéthyl)thiocarbamate.

9. Composé de formule (I) selon l'une quelconque des revendications 1 ou 2 qui est le chlorhydrate du 5-[(*3S*)-1,2-dihtiolan-3-yl]pentyl-N-(2-morpholinoéthyl)thiocarbamate.

10. Composé de formule (I) selon l'une quelconque des revendications 1 ou 2 qui est le chlorhydrate du 5-[(3*R*)-1,2-dithiolan-3-yl]pentyl-N-(2-morpholinoéthyl)thiocarbamate.

11. Composé de formule (I) selon l'une quelconque des revendications 1 ou 2 qui est le chlorhydrate du 5-(1,2-dithiolan-3-yl)pentyl-N-(3-morpholinopropyl)thiocarbamate.

12. Composé de formule (I) selon l'une quelconque des revendications 1 ou 3 qui est le chlorhydrate du 5-(1,2-dithiolan-4-yl)pentyl-N-(2-morpholinoéthyl)thiocarbamate.

13. Procédé de préparation des composés de formule (I) **caractérisé en ce qu'**on utilise comme produit de départ :
• *soit un composé de formule (II) :* 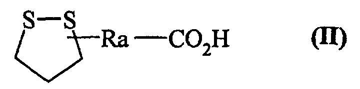 dans laquelle Ra est tel que défini dans la formule (I), composé de formule (II), que l'on traite en milieu basique avec un composé de formule (III): 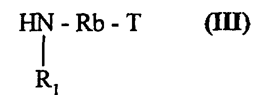 dans laquelle R₁, Rb et T sont tels que définis dans la formule (I), pour conduire aux composés de formule (IV): 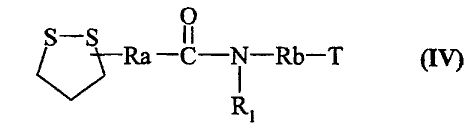 dans laquelle Ra, Rb, R₁ et T sont tels que définis précédemment, que l'on soumet au réactif de Lawesson, pour conduire aux composés de formule (I/a), cas particulier des composés de formule (I) : 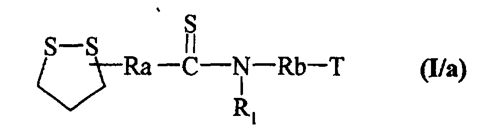 dans laquelle Ra, Rb, R₁ et T sont tels que définis précédemment,
• *soit un composé de formule (V) :* 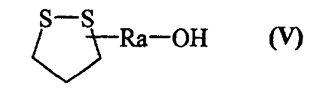 dans laquelle Ra a la même signification que dans la formule (I),
que l'on fait réagir, en présence de bis(tributylétain)oxyde, avec un thioisocyante de formule (VI) :
S = C = N - Rb - T (Vi)
dans laquelle Rb et T ont la même signification que dans la formule (I),
pour conduire aux composés de formule (I/b), cas particulier des composés de formule (I): 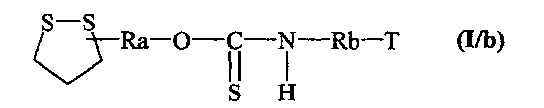 dans laquelle Ra, Rb, et T sont tels que définis précédemment,
composés de formule (I/b) que l'on traite, si on le souhaite, par un composé de formule (VII):
R₁ - X (VII)
dans laquelle R₁ est tel que défini dans la formule (I) et X représente un atome d'halogène,
pour conduire aux composés de formule (I/c), cas particulier des composés de formule (I): 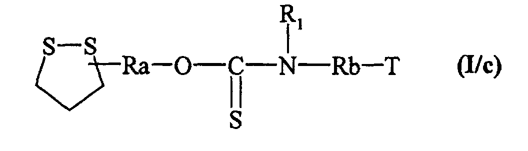 dans laquelle Ra, Rb, R₁ et T sont tels que définis précédemment,
les composés (I/a) à (I/c) formant l'ensemble des composés de l'invention, que l'on purifie, le cas échéant, selon une technique classique de purification, qui peuvent, si on le désire, être séparés en leurs différents isomères optiques selon une technique classique de séparation, et que l'on transforme, le cas échéant, en leurs sels d'addition à un acide ou à une base pharmaceutiquement acceptable.

14. Compositions pharmaceutiques contenant comme principe actif au moins un composé de formule (I) selon l'une quelconque des revendications 1 à 12, seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12 utiles en tant qu'agent antioxydant.

## Patentansprüche

1. Verbindungen der Formel (I): 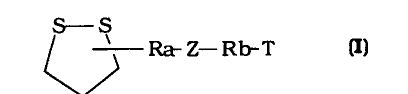 in der:
Ra eine geradkettige oder verzweigte (C₁-C₈)-Alkylengruppe bedeutet,
Rb eine Einfachbindung oder eine geradkettige oder verzweigte (C₁-C₆)-Alkylengruppe darstellt,
Z eine Gruppe:
• Thiocarbamat 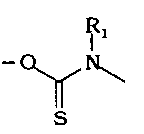 in der das Sauerstoffatom an die Gruppe Ra gebunden ist und R₁ ein Wasserstoffatom, eine geradkettige oder verzweigte (C₁-C₆)-Alkylgruppe, eine Arylgruppe oder eine geradkettige oder verzweigte Aryl-(C₁-C₆)-alkylgruppe bedeutet, oder
• Thioamid 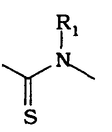 in der die Thiocarbonylgruppe an die Gruppe Ra gebunden ist und R₁ die oben angegebenen Bedeutungen besitzt, darstellt,
T eine Gruppe: 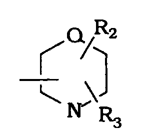 in der R₂ und R₃, die gleichartig oder verschieden sind, unabhängig voneinander eine Gruppe ausgewählt aus:
Wasserstoffatomen, geradkettigen oder verzweigten (C₁-C₆)-Alkylgruppen, Cycloalkylgruppen, geradkettigen oder verzweigten Cycloalkyl-(C₁-C₆)-alkylgruppen, Heterocycloalkylgruppen, geradkettigen oder verzweigten Heterocycloalkyl-(C₁-C₆)-alkylgruppen, Arylgruppen, geradkettigen oder verzweigten Aryl-(C₁-C₆)-alkylgruppen, Heteroarylgruppen, geradkettigen oder verzweigten Heteroaryl-(C₁-C₆)-alkylgruppen und geradkettigen oder verzweigten (C₁-C₆)-Aminoalkylgruppen, wobei der Aminorest gegebenenfalls durch eine oder zwei gleichartige oder verschiedene Gruppen ausgewählt aus geradkettigen oder verzweigten (C₁-C₆)-Alkylgruppen, Arylgruppen und geradkettigen oder verzweigten Aryl-(C₁-C₆)-alkylgruppen substituiert sein kann, bedeuten, darstellt,
deren optische Isomere sowie deren Additionssalze mit einer pharmazeutisch annehmbaren Säure oder Base,
mit der Maßgabe, daß:
- man unter Cycloalkyl eine mono- oder bicyclische, gesättigte oder ungesättigte Gruppe mit 3 bis 8 Kohlenstoffatomen versteht, wobei jede dieser Gruppen gegebenenfalls in gleichartiger oder verschiedener Weise substituiert ist durch eine oder mehrere Gruppen ausgewählt aus Halogenatomen, Hydroxygruppen, geradkettigen oder verzweigten (C₁-C₆)-Alkylgruppen, geradkettigen oder verzweigten (C₁-C₆)-Alkoxygruppen und Aminogruppen, die ihrerseits gegebenenfalls durch eine oder zwei gleichartige oder verschiedene, geradkettige oder verzweigte (C₁-C₆)-Alkylgruppen substituiert sein können, substituiert ist,
- man unter Heterocycloalkyl eine Cycloalkylgruppe zu verstehen ist, bei der ein oder zwei Kohlenstoffatome durch ein Heteroatom ersetzt ist, welches gleichartig oder verschiedenartig ausgewählt ist aus Stickstoff, Sauerstoff oder Schwefel,
- man unter Aryl eine Phenyl-, Naphthyl-, Indenyl-, Tetrahydronaphthyl-, Dihydronaphthyl- oder Indanylgruppe zu verstehen ist, wobei jede dieser Gruppen gegebenenfalls in gleichartiger oder verschiedener Weise substituiert ist durch eine oder mehrere Gruppen ausgewählt aus Halogenatomen, geradkettigen oder verzweigten (C₁-C₆)-Alkylgruppen, Hydroxygruppen, geradkettigen oder verzweigten (C₁-C₆)-Alkoxygruppen, Aminogruppen, geradkettigen oder verzweigten, Mono- oder Di-(C₁-C₆)-alkylaminogruppen, Carboxygruppen und geradkettigen oder verzweigten (C₁-C₆)-Alkoxycarbonylgruppen,
- und man unter Heteroaryl eine Arylgruppe versteht, bei der ein oder zwei Kohlenstoffatome durch ein Heteroatom ersetzt ist, welches in gleichartige oder unterschiedlicher Weise ausgewählt ist aus Sauerstoff, Stickstoff und Schwefel.

2. Verbindungen der Formel (I) nach Anspruch 1, **dadurch gekennzeichnet, daß** sie der Formel (IA) entsprechen: 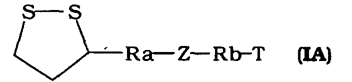 in der:
Ra eine geradkettige oder verzweigte (C₁-C₈)-Alkylengruppe bedeutet,
Rb eine Einfachbindung oder eine geradkettige oder verzweigte (C₁-C₆)-Alkylengruppe darstellt,
Z eine Gruppe:
• Thiocarbamat 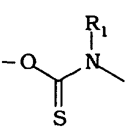 , in der das Sauerstoffatom an die Gruppe Ra gebunden ist und R₁ ein Wasserstoffatom, eine geradkettige oder verzweigte (C₁-C₆)-Alkylgruppe, eine Arylgruppe oder eine geradkettige oder verzweigte Aryl-(C₁-C₆)-alkylgruppe bedeutet, oder
• Thioamid 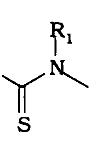 , in der die Thiocarbonylgruppe an die Gruppe Ra gebunden ist und R₁ die oben angegebenen Bedeutungen besitzt, darstellt,
T eine Gruppe: 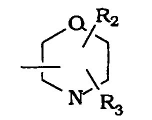 in der R₂ und R₃, die gleichartig oder verschieden sind, unabhängig voneinander eine Gruppe ausgewählt aus:
Wasserstoffatomen, geradkettigen oder verzweigten (C₁-C₆)-Alkylgruppen, Cycloalkylgruppen, geradkettigen oder verzweigten Cycloalkyl-(C₁-C₆)-alkylgruppen, Heterocycloalkylgruppen, geradkettigen oder verzweigten Heterocyclo-alkyl-(C₁-C₆)-alkylgruppen, Arylgruppen, geradkettigen oder verzweigten Aryl-(C₁-C₆)-alkylgruppen, Heteroarylgruppen, geradkettigen oder verzweigten Heteroaryl-(C₁-C₆)-alkylgruppen und geradkettigen oder verzweigten (C₁-C₆)-Aminoalkylgruppen, wobei der Aminorest gegebenenfalls durch eine oder zwei gleichartige oder verschiedene Gruppen ausgewählt aus geradkettigen oder verzweigten (C₁-C₆)-Alkylgruppen, Arylgruppen und geradkettigen oder verzweigten Aryl-(C₁-C₆)-alkylgruppen substituiert sein kann, bedeuten, darstellt,
deren optische Isomere sowie deren Additionssalze mit einer pharmazeutisch annehmbaren Säure oder Base.

3. Verbindungen der Formel (I) nach Anspruch 1, **dadurch gekennzeichnet, daß** sie der Formel (IB entsprechen: 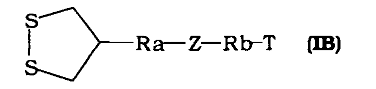
Ra eine geradkettige oder verzweigte (C₁-C₈)-Alkylengruppe bedeutet,
Rb eine Einfachbindung oder eine geradkettige oder verzweigte (C₁-C₆)-Alkylengruppe darstellt,
Z eine Gruppe:
• Thiocarbamat 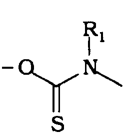 , in der das Sauerstoffatom an die Gruppe Ra gebunden ist und R₁ ein Wasserstoffatom, eine geradkettige oder verzweigte (C₁-C₆)-Alkylgruppe, eine Arylgruppe oder eine geradkettige oder verzweigte Aryl-(C₁-C₆)-alkylgruppe bedeutet, oder
• Thioamid 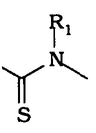 , in der die Thiocarbonylgruppe an die Gruppe Ra gebunden ist und R₁ die oben angegebenen Bedeutungen besitzt, darstellt,
T eine Gruppe: 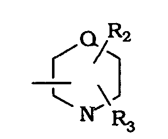 in der R₂ und R₃, die gleichartig oder verschieden sind, unabhängig voneinander eine Gruppe ausgewählt aus:
Wasserstoffatomen, geradkettigen oder verzweigten (C₁-C₆)-Alkylgruppen, Cycloalkylgruppen, geradkettigen oder verzweigten Cycloalkyl-(C₁-C₆)-alkylgruppen, Heterocycloalkylgruppen, geradkettigen oder verzweigten Heterocycloalkyl-(C₁-C₆)-alkylgruppen, Arylgruppen, geradkettigen oder verzweigten Aryl-(C₁-C₆)-alkylgruppen, Heteroarylgruppen, geradkettigen oder verzweigten Heteroaryl-(C₁-C₆)-alkylgruppen und geradkettigen oder verzweigten (C₁-C₆)-Aminoalkylgruppen, wobei der Aminorest gegebenenfalls durch eine oder zwei gleichartige oder verschiedene Gruppen ausgewählt aus geradkettigen oder verzweigten (C₁-C₆)-Alkylgruppen. Arylgruppen und geradkettigen oder verzweigten Aryl-(C₁-C₆)-alkylgruppen substituiert sein kann, bedeuten, darstellt.
deren optische Isomere sowie deren Additionssalze mit einer pharmazeutisch an nehmbaren Säure oder Base.

4. Verbindungen der Formel (I) nach einem der Ansprüche 1 bis 3, dadurcl gekennzeichnet, daß Z eine Gruppe 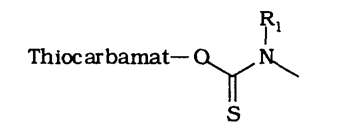 in der R₁ die bezüglich der Formel (I) angegebenen Bedeutungen besitzt, deren optische Isomere sowie deren Additionssalze mit einer pharmazeutisch annehmbaren Säure oder Base.

5. Verbindungen der Formel (I) nach einem der Ansprüche 1 bis 4, **dadurch gekennzeichnet, daß** Z eine Gruppe 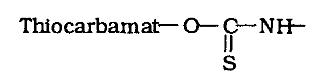 darstellt, deren optische Isomere sowie deren Additionssalze mit einer pharmazeutisch annehmbaren Säure oder Base.

6. Verbindungen der Formel (I) nach einem der Ansprüche 1 bis 3, **dadurch gekennzeichnet, daß** T eine Gruppe 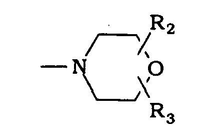 darstellt, worin R₂ und R₃ die bezüglich der Formel (I) angegebenen Bedeutungen besitzen, deren optische Isomere sowie deren Additionssalze mit einer pharmazeutisch annehmbaren Säure oder Base.

7. Verbindungen der Formel (I) nach Anspruch 6, **dadurch gekennzeichnet, daß** R₂ und R₃, die gleichartig oder verschieden sind, ein Wasserstoffatom bedeuten, deren optische Isomere sowie deren Additionssalze mit einer pharmazeutisch annehmbaren Säure oder Base.

8. Verbindung der Formel (I) nach einem der Ansprüche 1 oder 2, nämlich 5-(1,2-Dithiolan-3-yl)-pentyl-N-(2-morpholinoethyl)-thiocarbamat-Hydrochlorid.

9. Verbindung der Formel (I) nach einem der Ansprüche 1 oder 2, nämlich 5-[(*3S*)-1,2-Dithiolan-3-yl]-pentyl-N-(2-morpholinoethyl)-thiocarbamat-Hydrochlorid.

10. Verbindung der Formel (I) nach einem der Ansprüche 1 oder 2, nämlich 5-[(*3R*)-1,2-Dithiolan-3-yl]-pentyl-N-(2-morpholinoethyl)-thiocarbamat-Hydrochlorid.

11. Verbindung der Formel (I) nach einem der Ansprüche 1 oder 2, nämlich 5-(1,2-Dithiolan-3-yl)-pentyl-N-( 3-morpholinopropyl)-thiocarbamat-Hydrochlorid.

12. Verbindung der Formel (I) nach einem der Ansprüche 1 oder 3, nämlich 5-(1,2-Dithiolan-4-yl)-pentyl-N-(2-morpholinoethyl)-thiocarbamat-Hydrochlorid.

13. Verfahren zur Herstellung der Verbindungen der Formel (I), **dadurch gekennzeichnet, daß** man als Ausgangsprodukt:
• *entweder eine Verbindung der Formel (II)* verwendet: 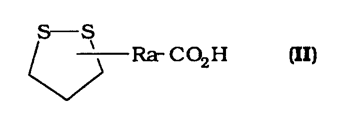 in der Ra die bezüglich der Formel (I) angegebenen Bedeutungen besitzt,
welche Verbindung der Formel (II) man in basischem Medium mit einer Verbindung der Formel (III) behandelt: 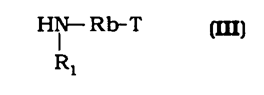 in der R₁, Rb und T die bezüglich der Formel (I) angegebenen Bedeutungen besitzen,
zur Bildung der Verbindungen der Formel (IV): 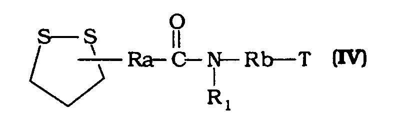 in der Ra, Rb, R₁ und T die oben angegebenen Bedeutungen besitzen,
welche man mit dem Lawesson-Reagens behandelt, zur Bildung der Verbindungen der Formel (I/a), einem Sonderfall der Verbindungen der Formel (I): 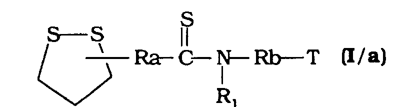 in der Ra, Rb, R₁ und T die oben angegebenen Bedeutungen besitzen,
• *oder eine Verbindung der Formel (V)* verwendet: 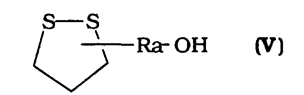 in der Ra die bezüglich der Formel (I) angegebenen Bedeutungen besitzt,
welche man in Gegenwart von Bis(tributylzinn)-oxid mit einem Thioisocyanat der Formel (VI) umsetzt:
S = C = N - Rb - T (VI)
in der Rb und T die bezüglich der Formel (I) angegebenen Bedeutungen besitzen,
zur Bildung der Verbindungen der Formel (I/b), einem Sonderfall der Verbindungen der Formel (I): 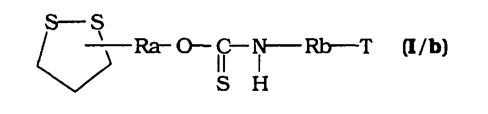 in der Ra, Rb und T die oben angegebenen Bedeutungen besitzen,
welche Verbindungen der Formel (I/b) man gewünschtenfalls mit einer Verbindung der Formel (VII) behandelt:
R₁ - X (VII)
in der R₁ die bezüglich der Formel (I) angegebenen Bedeutungen besitzt und X ein Halogenatom darstellt,
zur Bildung der Verbindungen der Formel (I/c), einem Sonderfall der Verbindungen der Formel (I): 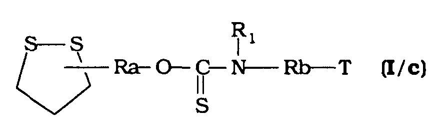 in der Ra, Rb, R₁ und T die oben angegebenen Bedeutungen besitzen,
welche Verbindungen (I/a) bis (I/c) die Gesamtheit der Verbindungen der Erfindung bilden, welche man gegebenenfalls mit Hilfe einer klassischen Reinigungsmethode reinigt, welche gewünschtenfalls mit einer üblichen Trennungsmethode in ihre verschiedenen optischen Isomeren getrennt werden können und welche man gegebenenfalls in ihre Additionssalze mit einer pharmazeutisch annehmbaren Säure oder Base umwandeln kann.

14. Pharmazeutische Zubereitungen, enthaltend als Wirkstoff mindestens eine Verbindung der Formel I) nach einem der Ansprüche 1 bis 12 allein oder in Kombination mit einem oder mehreren inerten, nichttoxischen, pharmazeutisch annehmbaren Trägermaterialien oder Bindemitteln.

15. Pharmazeutische Zubereitungen nach Anspruch 14, enthaltend mindestens einen Wirkstoff nach einem der Ansprüche 1 bis 12 als antioxidierende Mittel.

## Claims

1. Compounds of formula (I): 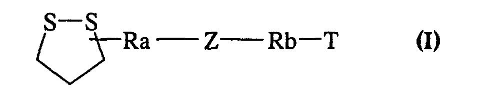 wherein:
Ra represents a linear or branched (C₁-C₈)alkylene group,
Rb represents a single bond or a linear or branched (C₁-C₆)alkylene group,
Z represents :
• a thiocarbamate group 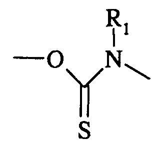 wherein the oxygen atom is bonded to the Ra group,
and R₁ represents a hydrogen atom, a linear or branched (C₁-C₆)alkyl group, a aryl group or an aryl-(C₁-C₆)alkyl group in which the alkyl moiety is linear branched,
or
• a thioamide group 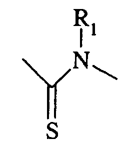 wherein the thiocarbonyl group is bonded to the Ra group,
and R₁ is as defined hereinbefore,
T represents a group: 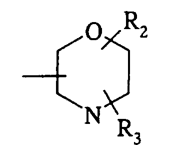 wherein R₂ and R₃, which may be identical or different, each independently of the other represents a group selected from :
a hydrogen atom, a linear or branched (C₁-C₆)alkyl group, a cycloalkyl group, a cycloalkyl-(C₁-C₆)alkyl group in which the alkyl moiety is linear or branched, a heterocycloalkyl group, a heterocycloalkyl-(C₁-C₆)alkyl group in which the alkyl moiety is linear or branched, an aryl group, an aryl-(C₁-C₆)alkyl group in which the alkyl moiety is linear or branched, a heteroaryl group, a heteroaryl-(C₁-C₆)alkyl group in which the alkyl moiety is linear or branched, and an amino-(C₁-C₆)alkyl group in which the alkyl moiety is linear or branched, the amino moiety being optionally substituted by one or two identical or different groups selected from linear or branched (C₁-C₆)alkyl, aryl and aryl-(C₁-C₆)alkyl in which the alkyl moiety is linear or branched,
their optical isomers, and addition salts thereof with a pharmaceutically acceptable acid or base,
it being understood that:
- "cycloalkyl" is understood to mean a mono- or bi-cyclic, saturated or unsaturated group having from 3 to 8 carbon atoms, each of those groups being optionally substituted by one or more identical or different groups selected from a halogen atom, a hydroxy group, a linear or branched (C₁-C₆)alkyl group, a linear or branched (C₁-C₆)alkoxy group, and an amino group (amino itself optionally substituted by one or two identical or different linear or branched (C₁-C₆)alkyl groups),
- "heterocycloalkyl" is understood to mean a cycloalkyl group in which one or two carbon atoms have been replaced by a hetero atom selected, identically or differently, from nitrogen, oxygen and sulphur,
- "aryl" is understood to mean a phenyl, naphthyl, indenyl, tetrahydronaphthyl, dihydronaphthyl or indanyl group, each of those groups being optionally substituted by one or more identical or different groups selected from a halogen atom, a linear or branched (C₁-C₆)alkyl group, a hydroxy group, a linear or branched (C₁-C₆)alkoxy group, an amino group, a mono- or di-(C₁-C₆)alkyl-amino group in which alkyl is linear or branched, a carboxy group, and a linear or branched (C₁-C₆)alkoxycarbonyl group, and
- "heteroaryl" is understood to mean an aryl group in which one or two carbon atoms have been replaced by a hetero atom selected, identically or differently, from oxygen, nitrogen and sulphur.

2. Compounds of formula (I) according to claim 1 **characterised in that** they represent a compound of formula (IA): 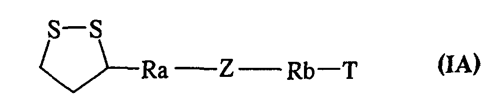 wherein:
Ra represents a linear or branched (C₁-C₈)alkylene group,
Rb represents a single bond or a linear or branched (C₁-C₆)alkylene group,
Z represents :
• a thiocarbamate group 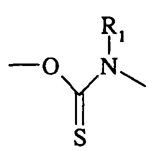 wherein the oxygen atom is bonded to the Ra group,
and R₁ represents a hydrogen atom, a linear or branched (C₁-C₆)alkyl group, an aryl group or an aryl-(C₁-C₆)alkyl group in which the alkyl moiety is linear or branched,
or
• a thioamide group 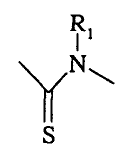 wherein the thiocarbonyl group is bonded to the Ra group,
and R₁ is as defined hereinbefore,
T represents a group: 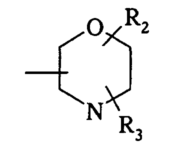 wherein R₂ and R₃, which may be identical or different, each independently of the other represents a group selected from :
a hydrogen atom, a linear or branched (C₁-C₆)alkyl group, a cycloalkyl group, a cycloalkyl-(C₁-C₆)alkyl group in which the alkyl moiety is linear or branched, a heterocycloalkyl group, a heterocycloalkyl-(C₁-C₆)alkyl group in which the alkyl moiety is linear or branched, an aryl group, an aryl-(C₁-C₆)alkyl group in which the alkyl moiety is linear or branched, a heteroaryl group, a heteroaryl-(C₁-C₆)alkyl group in which the alkyl moiety is linear or branched, and an amino-(C₁-C₆)alkyl group in which the alkyl moiety is linear or branched, the amino moiety being optionally substituted by one or two identical or different groups selected from linear or branched (C₁-C₆)alkyl, aryl and aryl-(C₁-C₆)alkyl in which the alkyl moiety is linear or branched,
their optical isomers, and addition salts thereof with a pharmaceutically acceptable acid or base.

3. Compounds of formula (I) according to claim 1 **characterised in that** they represent a compound of formula (IB): 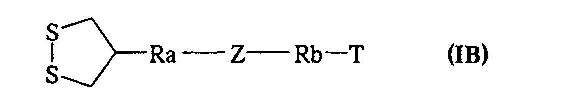 wherein:
Ra represents a linear or branched (C₁-C₈)alkylene group,
Rb represents a single bond or a linear or branched (C₁-C₆)alkylene group,
Z represents :
• a thiocarbamate group 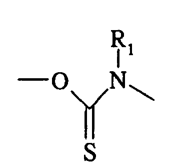 wherein the oxygen atom is bonded to the Ra group,
and R₁ represents a hydrogen atom, a linear or branched (C₁-C₆)alkyl group, an aryl group or an aryl-(C₁-C₆)alkyl group in which the alkyl moiety is linear or branched,
or
• a thioamide group 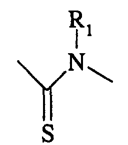 wherein the thiocarbonyl group is bonded to the Ra group,
and R₁ is as defined hereinbefore,
T represents a group: 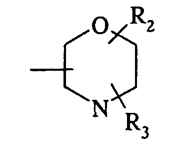 wherein R₂ and R₃, which may be identical or different, each independently of the other represents a group selected from :
a hydrogen atom, a linear or branched (C₁-C₆)alkyl group, a cycloalkyl group, a cycloalkyl-(C₁-C₆)alkyl group in which the alkyl moiety is linear or branched, a heterocycloalkyl group, a heterocycloalkyl-(C₁-C₆)alkyl group in which the alkyl moiety is linear or branched, an aryl group, an aryl-(C₁-C₆)alkyl group in which the alkyl moiety is linear or branched, a heteroaryl group, a heteroaryl-(C₁-C₆)alkyl group in which the alkyl moiety is linear or branched, and an amino-(C₁-C₆)alkyl group in which the alkyl moiety is linear or branched, the amino moiety being optionally substituted by one or two identical or different groups selected from linear or branched (C₁-C₆)alkyl, aryl and aryl-(C₁-C₆)alkyl in which the alkyl moiety is linear or branched,
their optical isomers, and addition salts thereof with a pharmaceutically acceptable acid or base.

4. Compounds of formula (I) according to any one of claims 1 to 3 **characterised in that** Z represents a thiocarbamate group 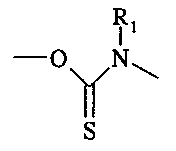 wherein R₁ is as defined for formula (I),
their optical isomers, and addition salts thereof with a pharmaceutically acceptable acid or base.

5. Compounds of formula (I) according to any one of claims 1 to 4 **characterised in that** Z represents a thiocarbamate group 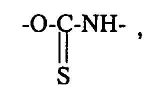 their optical isomers, and addition salts thereof with a pharmaceutically acceptable acid or base.

6. Compounds of formula (I) according to any one of claims 1 to 3 **characterised in that** T represents the group 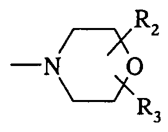 wherein R₂ and R₃ are as defined for formula (I), their optical isomers, and addition salts thereof with a pharmaceutically acceptable acid or base.

7. Compounds of formula (I) according to claim 6 **characterised in that** R₂ and R₃, which are identical, represent a hydrogen atom, their optical isomers and addition salts thereof with a pharmaceutically acceptable acid or base.

8. Compound of formula (I) according to either one of claims 1 or 2 which is 5-(1,2-dithiolan-3-yl)pentyl N-(2-morpholinoethyl)thiocarbamate hydrochloride.

9. Compound of formula (I) according to either one of claims 1 or 2 which is 5-[(*3S*)-1,2-dithiolan-3-yl]pentyl N-(2-morpholinoethyl)thiocarbamate hydrochloride.

10. Compound of formula (I) according to either one of claims 1 or 2 which is 5-[(3*R*)-1,2-dithiolan-3-yl]pentyl N-(2-morpholinoethyl)thiocarbamate hydrochloride.

11. Compound of formula (I) according to either one of claims 1 or 2 which is 5-(1,2-dithiolan-3-yl)pentyl N-(3-morpholinopropyl)thiocarbamate hydrochloride.

12. Compound of formula (I) according to either one of claims 1 or 3 which is 5-(1,2-dithiolan-4-yl)pentyl N-(2-morpholinoethyl)thiocarbamate hydrochloride.

13. Process for the preparation of compounds of formula (I) **characterised in that** there is used as starting material :
• *a compound of formula (II) :* 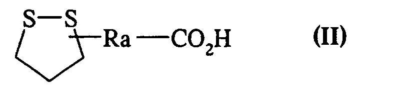 wherein Ra is as defined for formula (I), which compound of formula (II) is treated in a basic medium with a compound of formula (III): 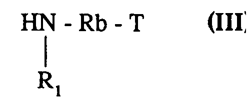 wherein R₁, Rb and T are as defined for formula (I),
to yield compounds of formula (IV) : 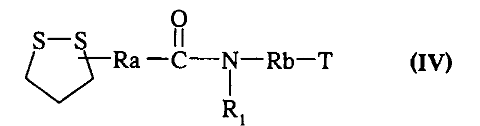 wherein Ra, Rb, R₁ and T are as defined hereinbefore,
which is subjected to Lawesson's reagent, to yield compounds of formula (I/a), a particular case of the compounds of formula (I): 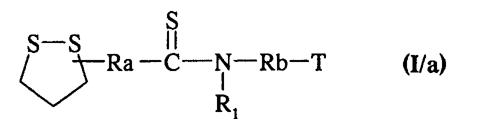 wherein Ra, Rb, R₁ and T are as defined hereinbefore,
or
• *a compound of formula (V):* 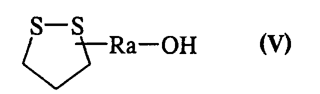 wherein Ra is as defined for formula (I),
which is reacted, in the presence of bis(tributyltin) oxide, with a thioisocyanate of formula (VI):
S = C = N - Rb - T (VI)
wherein Rb and T are as defined for formula (I),
to yield compounds of formula (I/b), a particular case of the compounds of formula (I): 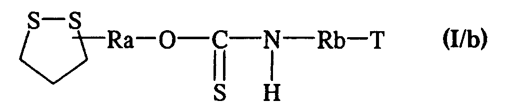 wherein Ra, Rb and T are as defined hereinbefore,
which compounds of formula (I/b) are treated, if desired, with a compound of formula (VII) :
R₁ - X (VII)
wherein R₁ is as defined for formula (I) and X represents a halogen atom,
to yield compounds of formula (I/c), a particular case of the compounds of formula (I): 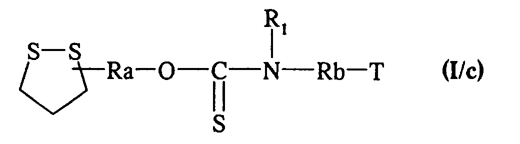 wherein Ra, Rb, R₁ and T are as defined hereinbefore,
which compounds (I/a) to (I/c) constitute the totality of the compounds of the invention, are purified, if necessary, according to a conventional purification technique, may be separated, if desired, into their different optical isomers according to a conventional separation technique, and are converted, where appropriate, into addition salts thereof with a pharmaceutically acceptable acid or base.

14. Pharmaceutical compositions comprising as active ingredient at least one compound of formula (I) according to any one of claims 1 to 12, on its own or in combination with one or more inert, non-toxic, pharmaceutically acceptable excipients or carriers.

15. Pharmaceutical compositions according to claim 14 comprising at least one active ingredient according to any one of claims 1 to 12 for use as an anti-oxidation agent.
